# EUROPÄISCHE PATENTSCHRIFT

(11) **EP 1 839 640 B1**
(45) Veröffentlichungstag und Bekanntmachung des Hinweises auf die Patenterteilung: **03.11.2010**
(21) Anmeldenummer: 07004865.7
(22) Anmeldetag: 09.03.2007
(51) Int. Cl.: A61K 6/083

(54) **Härtbare, dentale Massen**
Curable dental materials
Masses dentaires durcissables

(30) Priorität: 30.03.2006 DE 102006014772
(43) Veröffentlichungstag der Anmeldung: 03.10.2007
(73) Patentinhaber: VOCO GmbH, 27472 Cuxhaven (DE)
(72) Erfinder: Christmann, Lars, Dr., 27474 Cuxhaven (DE); Plaumann, Manfred Thomas, 27472 Cuxhaven (DE)

(56) Entgegenhaltungen:
- EP-A- 0 951 896
- WO-A-02/092021
- WO-A-02/092023

## Beschreibung

Die vorliegende Erfindung betrifft kunststoffverstärkte, chemisch härtende Glasionomerzemente zur Befestigung dentaler und kieferorthopädischer Vorrichtungen, zur Überkappung der Pulpa als Unterfüllung unter allen Füllungsmaterialien sowie als Füllungsmaterial restaurationsbedürftiger Zähne. Kunststoffmodifizierte Glasionomerzemente sind aus dem Stand der Technik bekannt und werden durch Beigabe von Kunststoffkomponenten zu konventionellen Glasionomerzementen dargestellt.

Ursprüngliches Ziel bei der Entwicklung kunststoffmodifizierter Glasionomerzemente war die Verbesserung der physikalischen Eigenschaften und eine Vereinfachung der Handhabung herkömmlicher Glasionomerzemente.

Konventionelle Glasionomerzemente weisen gegenüber Kompositmaterialien niedrigere Abrasions- und Zugfestigkeiten, ein schlechteres ästhetisches Erscheinungsbild sowie eine hohe Anfälligkeit für Verarbeitungsfehler auf. Insbesondere sind Glasionomerzemente während ihrer Anwendung sehr empfindlich gegenüber Feuchtigkeit: Gelangt der Zement in einer frühen Stufe seiner Aushärtung in Kontakt mit Feuchtigkeit, wie mit Speichel, dann wird der Fortgang der Abbindung gestört, so daß die physikalischen Eigenschaften des gehärteten Zementes verschlechtert sind. Diese Nachteile konnten durch Zugabe von Kompositelementen zu den Glasionomerzementen in bestimmten Maßen behoben werden.

In den ersten Hybridmaterialien wurde lediglich ein geringer Anteil der Menge des Wassers aus dem herkömmlichen Glasionomersystem durch wasserlösliche und radikalisch vernetzbare Monomere wie Hydroxyethylmethacrylat (HEMA) und Polyethylenglykoldimethacrylat ersetzt. In Gegenwart eines chemischen Redoxsystems zur Initiierung einer Radikalkettenreaktion, wie es beispielsweise das System "Benzoylperoxid/Sulfinsäuresalz" darstellt, kann das Hybrid-System dann gemäß einem dualen Aushärtungsmechanismus vernetzen.

Zunächst wird hierbei die redoxinitiierte Vernetzung der ethylenisch ungesättigten Verbindungen einsetzen. Durch die beginnende Verfestigung des Materials wird das Glasionomersystem feuchtigkeitsunempfindlicher und kann dann, kinetisch langsamer, ungestört weiter durch die Säure-Base Reaktion des Glasionomers abbinden. Bei kunststoffmodifizierten Glasionomerzementen liegt der kunststoffmodifizierte Anteil im Gesamtsystem quantitativ in sehr viel geringerer Menge vor als der Glasionomerzementanteil. Die Eigenschaften dieser hybriden Systeme werden somit in erster Linie vom eingesetzten Glasionomer dominiert.

Kunststoffmodifizierte Glasionomerzemente sind zweikomponentig formuliert und werden zum Einsatz als Befestigungszement, Unterfüllungsmaterial oder als Füllungsmaterial selbst in der Form Pulver/Flüssigkeit angewendet.

Die Aushärtereaktion wird in dem Moment eingeleitet, in dem Pulver und

Flüssigkeit miteinander vermischt werden und erfolgt in der GIZ-Reaktion formal als Neutralisation der Säuregruppen des wasserlöslichen Polymers mit dem pulverförmigen basischen Silikatglas. Das Glas kann beispielsweise ein Strontiumaluminiumfluorsilikatglas sein.

Im ersten Schritt der Glasionomerabbindereaktion attackieren die Protonen der Polysäure unter den wäßrigen Bedingungen die Oberfläche der Glaspartikel und setzen Strontium- und Aluminiumionen frei. Die Metallionen kombinieren mit den Carboxylsäuregruppen der Polyacrylsäure unter Bildung einer Polysäuresalzmatrix. Mit fortschreitender Freisetzung der Kationen wird das Glasgitter unter Bildung von Kieselsäure aufgebrochen, wobei die Säure auf der Oberfläche der Glasteilchen zu einem Kieselsäurehydrogel polymerisiert. Die Ionenkonzentration in der wäßrigen Phase erhöht sich mit der Umwandlung von Polysäure zu Polysalz, der pH-Wert steigt und die Viskosität der Mischung erhöht sich.

Mit der Abbindung ändert sich ebenfalls der Koordinationszustand des Aluminiums von 4 auf 6. Während das Aluminiumion im Glas von 4 Sauerstoffatomen umgeben ist, wird das Ion im abgebundenen Zustand von 6 Atomgruppen koordiniert, die dann Wasser, Fluoridionen, Carboxylgruppen oder andere anionische Spezies sein können.

Bei einer bestimmten Ionenkonzentration und einem bestimmten pH-Wert beginnt das Polysalz auszufallen und die Gelierung der Zementmischung setzt ein. Der Zement wird nun fest. Selbst nach dem Abbindevorgang läuft die Polysalzausfällung weiter bis alle Ionen in nicht gelöster Form vorliegen. Man vermutet, daß der Beginn der Abbindung auf dem Ausfällen von Sr-Polysalz beruht. Diese frühe Reaktionsphase ist instabil und wird auch "primäre Härtung" genannt. Der nachfolgende Härtungsprozeß beruht auf der langsameren Bildung von Aluminiumpolysalz, das schließlich die dominierende Phase der Glasionomermatrix bildet. Dieses fortgeschrittene Stadium, das durch die Koordination der Al-lonen mit den Carboxylgruppen der Polyanionen zu Al-Polyalkenoat gekennzeichnet ist, ist stabil und wird auch "sekundäre Härtung" genannt. Die Aluminiumionen werden wahrscheinlich in Form komplexer Oxyanionen mit mehreren Aluminiumatomen freigesetzt und entsprechen strukturell zunächst weitestgehend der Struktur innerhalb der Gläser vor dem Säureangriff. Durch die Bildung des Al-Polyalkenoats bedingt, wird Wasser aus einigen Hydratationsbereichen verdrängt. Dies führt zu ionischen Brückenbindungen der Polysäureketten und erklärt die Festigkeit des Materials.

Über die genaue Abfolge der lonenfreisetzung während der Abbindephase herrscht jedoch immer noch kein allgemeiner Konsens. Mehrere Untersuchungen legen den Schluß nahe, daß auch schon zu Beginn der Aushärtung Aluminiumionen abgegeben werden, die jedoch nicht direkt einer weiteren Reaktion zugänglich sind und zunächst in Form kondensierter Spezies wie [Al₁₃O₄(OH)₂₄(H₂O₁₂]⁷⁺ auftreten und reaktive Aluminiumionen erst in einem zweiten Schritt langsam abgeben.

Es ist bekannt, daß im abgebundenen Glasionomerzement noch bis zu einem Jahr langsame strukturelle Änderungen stattfinden, die sich in verbesserter Transluzenz und Festigkeit des Materials äußern. Man nimmt an, daß diese Eigenschaftsverbesserungen auf Zunahme der Menge des gebundenen Wassers zurückzuführen sind. Dies könnte dann zu einer erhöhten Hydratisierung der Metall-Salz Bindungen führen.

Die früher vorherrschende Annahme, daß ein abgebundener Polyalkenoatzement eine Mischung einfacher, durch Glaspartikel verstärkter Metallpolyacrylatsalze ist, kann nicht mehr uneingeschränkt aufrechterhalten werden. So zeigten Untersuchungen, daß sowohl Silizium als auch Phosphor durchgängig in der Zementmatrix auftreten. Dies legt die Vermutung nahe, daß beide Elemente ein eigenes anorganisches Netzwerk ausbilden, das die Metallpolyacrylatstruktur durchdringt und so mit die mechanischen Werte des Zements kontrolliert. Studien belegen, daß die anorganischen Spezies in der Tat einen wesentlichen Beitrag zur Bildung der Zementmatrix wahrnehmen. Diese bedeutende Feststellung läßt die "Nachabbindephase" in neuem Licht erscheinen und erklärt ebenfalls, warum eine komplexe Glaszusammensetzung für den Abbindevorgang des Glasionomers notwendig ist und eine einfache Mischung von Ca/Sr Ionen mit Al Ionen in Reaktion mit den Polysäuren nicht zu einem Material mit auch nur halbwegs befriedigenden Eigenschaften führt. Die Ausbildung der anorganischen Phase macht darüber hinaus plausibel, warum Glasionomerzemente weniger hydrolyseanfällig als Zinkcarboxylatzemente sind, obwohl die Assoziation der Aluminium- und Strontiumionen mit organischen Säuren sehr viel schwächer im Vergleich zur analogen Neutralisation in Zinkcarboxylatzementen ist.

Ein weiterer bedeutsamer Punkt während der Abbindung scheint die Konformationsänderung der Polyacrylsäure zu sein. Studien belegen, daß die Polyacrylsäure als Elekrolyt mit zunehmender Neutralisation und somit steigendem pH-Wert eine sogenannte "Windungsexpansion" (coil expansion) durchläuft. Während die Struktur der Säure bei niedrigem pH-Wert eine allseits zufällig gewundene Konformation aufweist, ändert sich dieser Status bei steigendem pH-Wert und die Konformation nimmt eine starre, stabförmige Struktur an. Diese Konformationsänderung geht einher mit zunehmender Viskosität der Mischung und Werten höherer Dichte der Zusammensetzung. Diese nunmehr hoch geordnete Struktur mit ausgeprägtem Domänencharakter scheint ebenfalls für Festigkeit und Hydrolysenbeständigkeit des resultierenden Materials verantwortlich zu sein.

Experimentelle Untersuchungen zeigen weiterhin, daß der Wert maximaler Biegefestigkeit eines Zements unabhängig vom Molekulargewicht der Polyacrylsäure ist, falls die Säure in einer bestimmten Konzentration vorliegt. Dieser Befund führt zu der Schlußfolgerung, daß die Biegefestigkeit in erster Linie von der Dichte der Carboxylgruppen und nicht von der Länge der Polymerkette abhängt. Dies bedeutet, daß eine hohe Biegefestigkeit entweder durch eine hohe Konzentration kleiner Polysäureketten oder durch geringere Konzentration langer Polysäureketten zu erreichen ist.

Parallel zum GIZ-Abbindemechanismus läuft in den kunststoffmodifizierten Glasionomerzementen in einer kinetisch schneller abgeschlossenen Reaktion die redoxinitiierte Polymerisation der ungesättigten Verbindungen ab. In der Regel handelt es sich bei allen in der Dentalchemie im sauren Milieu eingesetzten Redoxsystemen um Varianten des sogenannten "Bredereck Systems", das in der Patentliteratur (DE 1495520 "Verfahren zur Substanzpolymerisation") schon 1964 von Bredereck (Deutsche Gold- und Silberscheideanstalt) angemeldet wurde. Untersuchungen zur redoxinitiierten Vernetzung ungesättigter Verbindungen begannen in der Arbeitsgruppe von Bredereck bereits in den 40er und 50er Jahren (US 2,779,751 "Polymerization process using sulfone-containing accelerators", 1952 / US 2,776,952 "Improvements in the production of polymerization products using halogen compounds to increase the effectiveness of sulfonpolymerization catalysts", 1952 / US 2,846,418 "Process for the production of polymerization products", 1953 / US 2,935,489 "Process for polymerizing ethylenically unsaturated organic compounds with the aid of hydroxylamine derivatives", 1956 /US 2,894,932 "Adhesive composition comprising an acrylate monomer, an unsaturated polyester and polyvinylmethylether", 1957).

In der Regel benutzt man in der Dentalchemie für chemisch härtende Massen das System "Benzoylperoxid/aromatisches tertiäres Amin". Soll dieses System jedoch im sauren Milieu eingesetzt werden, wie es bei kunststoffverstärkten Glasionomerzementen vorliegt, versagt die Wirksamkeit der Redoxpartner. Kommt das aromatische tertiäre Amin in Kontakt mit leichten oder starken Säuren, wird die Verbindung protoniert und verliert sofort ihre Funktionstüchtigkeit als Reduktionsmittel. Darüberhinaus wird das Benzoylperoxid unter sauren Bedingungen leicht abgebaut. Dies sind die Gründe, warum man für chemisch härtende dentale Zusammensetzungen schon früh auf die "Bredereck"-Systeme zurückgriff.

Seit den Anfängen in der Erforschung der unter sauren Bedingungen stabilen Redoxsysteme wurden diese immer konsequenter erweitert.

Die DE 101 24 029 A1 offenbart ein Initiatorsystem für saure Dentalformulierungen, das umfaßt, a.) 14,9 bis 50 Gew.-% einer Barbitursäure oder Thiobarbitursäure bzw. einem Barbitursäure- oder Thiobarbitursäurederivat, b.) 30 bis 75 Gew.-% einer Peroxodisulfatverbindung und/oder Peroxodiphosphatverbindung, c.) 10 bis 35 Gew.-% einer Sulfinsäureverbindung und d.) 0,1 bis 5 Gew.-% einer Kupferverbindung.

Das Initiatorsystem auf Basis einer zweikomponentigen Pulver/Flüssigkeitformulierung soll zur Herstellung und Anwendung von haftvermittelnden Stoffen, dentalen Befestigungsmassen und Zahnfüllmaterialien mit variablem Füllstoffgehalt geeignet sein und eine hohe Haftung des polymerisierten Materials an der Zahnhartsubstanz bewirken. Reduktions- und Oxidationsmittel sind Bestandteile der Pulverkomponente, während der Schwermetallkomplex im Flüssigteil untergebracht ist.

Die DE 197 57 277 A1 beschreibt ebenfalls ein dentales Initiatorsystem zur Aushärtung saurer, zweikomponentiger Massen auf der Basis Pulver/Flüssigkeit. Der Initiator setzt sich aus einem Kupfersalz, einer Sulfinsäureverbindung und einem Barbitur- bzw. Thiobarbitursäurederivat zusammen. Das Initiatorsystem soll ebenfalls zur Herstellung und Anwendung von haftvermittelnden Stoffen, dentalen Befestigungsmassen und Zahnfüllmaterialien mit variablem Füllstoffgehalt geeignet sein und soll eine ausreichende Haftung des polymerisierten Materials an der Zahnhartsubstanz sowie eine schnelle und anwendungsfreundliche Aushärtungszeit bewirken. Auch in diesem Dokument sind Reduktions- und Oxidationsmittel Bestandteile der Pulverkomponente, während das Kupfersalz Bestandteil der flüssigen Phase ist.

Die DE 100 17 188 A1 beansprucht zweikomponentige Dentalmassen mit niedriger Abbindetemperatur, wobei die Komponente I: a.) 0,1 bis 20 Gew.-% mindestens eines Vinylethers, b.) 10 bis 89,9 Gew.-% mindestens eines ethylenisch ungesättigten Monomers, das kein Vinylether ist, c.) 0,001 bis 5 Gew.-% mindestens eines Beschleunigers, d.) 9,999 bis 89.899 Gew.-% Füllstoffe, Thixotropie-Hilfsmittel und andere Hilfsstoffe und Komponente 11: e.) 0,1 bis 20 Gew.-% mindestens eines Barbitursäurederivates und/oder Malonylsulfamides, das die radikalische Polymerisation auslösen kann, f.) 0 bis 89,9 Gew.-% Füllstoffe, Thixotropie-Hilfsmittel, Verzögerer und anderer Hilfsstoffe, g.) 10 bis 80 Gew.-% übliche Weichmacher enthält.

Als Beschleuniger gemäß Bestandteil c.) ist der Einsatz von Schwermetallverbindungen und insbesondere von Kupferkomplexen sowie ionogen gebundenen Halogenen oder Pseudohalogenen wie organische Ammoniumchloride vorgesehen. In den Beispielen wird die 1-Benzyl-5-phenylbarbitursäure als Reduktionsmittel, das β-(Phenylethyl)-dibutylammoniumchlorid und der (Bis-(1-Phenylpentan-1,3-dionato)-kupfer(II)) Komplex als Beschleuniger in dem Paste-Paste System eingesetzt. Das Reduktionsmittel ist Teil der einen Paste, während Schwermetallkomplex und ionogene Halogenverbindung Teile der anderen Paste sind. Diese erfindungsgemäße Zusammensetzung härtet ohne Peroxid.

In der DE 199 28 238 A1 wird eine polymerisierbare Dentalmasse vorgeschlagen, die enthält a.) 10 bis 98,999 Gew.-% mindestens eines bi- oder höherfunktionellen ethylenisch ungesättigten Monomers, b.) 0 bis 88,999 Gew.% mindestens eines monofunktionellen ethylenisch ungesättigten Monomers, c.) 0 bis 5 Gew.-% eines Beschleunigers, d.) 0,001 bis 5 Gew.-% eines Redoxinitiatorsystems, das die radikalische Polymerisation auslösen kann, e.) 0 bis 88,999 Gew.-% Füllstoffe, Thixotropie-Hilfsmittel, Verzögerer und anderer Hilfsstoffe, f.) 1 bis 30 Gew.-% eines üblichen Weichmachers und dadurch gekennzeichnet ist, daß das Redoxinitiatorsystem ein Barbitursäurederivat und/oder ein Malonylsulfamid und ein organisches Peroxid, ausgewählt aus der Gruppe der ein- oder mehrfunktionellen Carbonsäureperoxyester, umfaßt.

Auch hier wird in den Beispielen die 1-Benzyl-5-phenylbarbitursäure als Reduktionsmittel, das β-(Phenylethyl)-dibutylammoniumchlorid und der (Bis-(1-Phenylpentan-1,3-dionato)-kupfer(II)) Komplex als Beschleuniger in dem Paste-Paste System eingesetzt. Zum Reduktionsmittel kann hier der 3,5,5-Trimethylhexansäure-tert. butylperoxyester als Oxidationsmittel zugefügt werden, ohne daß das System seine Stabilität verliert. Die ionogene Halogenverbindung und der Schwermetallkomplex als Teile des Redoxsystems sind Bestandteile der anderen Paste. Diese Dentalmassen sollen sich als Füllungsmaterialien, Stumpfaufbaumaterialien, Befestigungszemente sowie als provisorische Kronen- und Brückenmaterialien eignen.

Die EP 0 732 098 stellt eine dentale, polymerisierbare Zusammensetzung in Form eines Pulver/Flüssigkeitsystems bereit, die ein Peroxid, das sich innerhalb von 10 Stunden bei einer Temperatur von mindestens 75°C zu höchstens 50 Gew.-% zersetzt, einen Protonendonor, eine metallorganische Verbindung, ein polymerisierbares Monomer und vorzugsweise zusätzlich ein Amin enthält. Die Pulverkomponente umfaßt den Kupferthioharnstoffkomplex, Barbitur- oder Ascorbinsäure sowie Füllstoffe, während die flüssige Phase die Monomeren, Füllstoffe, das tert.-Butyl-peroxy-(3,5,5-trimethylhexanoat) sowie das N,N-Dihydroxyethyl-p-toluidin aufweist. Die Zusmmensetzung soll schnell abbinden und eine ausreichende Lagerstabilität besitzen. Sie wird als Versiegelungsmaterial oder Klebstoff eingesetzt.

Die DE 44 02 100 beschreibt eine kalt polymerisierende Zahnzusammensetzung als provisorisches Kronen- und Brückenmaterial vom Pulver/Flüssigkeitstyp, die im Pulverteil das Kupfer-(II)acetylacetonat, Füllstoffe sowie die 1-Benzyl-5-phenylbarbitursäure enthält und in der flüssigen Phase das Dilauryldimethylammoniumchlorid, die Monomeren und das N,N-Dihydroxypropyl-p-toluidin aufweist. Diese Erfindung soll inhomogenes Aushärten, ein Gelbwerden des gehärteten Materials sowie die Erzeugung innerer Luftblasen beim Aufstreichen verhindern.

Die EP 1 387 657 offenbart selbstadhäsive Dentalmaterialien. In dieser Anmeldung wird ein dualhärtender Befestigungszement als zweikomponentiges Pulver/Flüssigkeit System beschrieben. Die Pulverkomponente umfaßt ein silanisiertes Strontiumaluminiumfluorosilikatglas, Kalziumhydroxid, Natriumtoluolsulfinat, 1,3-Dimethyl-5-phenylbarbitursäure, Natriumperoxodisulfat und pyrogene Kieselsäure. Die Flüssigkeit weist 1,3 Glyzerindimethacrylatphosphat, propoxyliertes Bisphenol-A-dimethacrylat, Triethylenglykoldimethacrylat, Campherchinon und Kupfer(II)acetat auf.

Das System stellt einen sogenannten "Komposit-Befestigungszement" dar und vernetzt radikalisch sowohl durch Licht als auch über ein Redoxsystem. Dieser Zement soll ohne Vorbehandlung wie Ätzen, Primen, Bonden oder Konditionieren angewendet werden. Er soll Haftwerte von mindestens 2,0 MPa an Rinderdentin aufweisen.

Alle oben beschriebenen Zusammensetzungen sind vom Komposittyp, somit hydrophob und wasserfrei.

Demgegenüber stellt die EP 0 323 120 A2 einen photohärtbaren lonomerzement für den Einsatz als dentales Adhäsiv, Füllungsmaterial, Unterfüllungsmaterial und Befestigungszement vor.

Die DE 3941629 A1 beansprucht ebenfalls eine Dentalglasionomerzementmasse, die ein Fluoraluminosilikatglaspulver, ein Polymer einer α,β-ungesättigten Carbonsäure, eine polymerisierbare ungesättigte organische Verbindung, einen Polymerisationskatalysator, Wasser, ein Tensid sowie ein Reduktionsmittel umfaßt. Sämtliche Beispiele des Dokuments beschreiben photohärtbare Systeme.

Die EP 1 269 968 A1 offenbart einen dentalen Zement, der umfaßt a.) ein polymerisierbares Monomer mit Säuregruppe, b.) eine Polyalkensäure, c.) einen Ionen abgebenden Füllstoff, d.) ein polymerisierbares Monomer ohne Säurefunktion, e.) Wasser, f.) ein Peroxid, g.) ein Salz einer aromatischen Sulfinsäure und h.) ein aromatisches sek. oder tert. Amin. Oxidations- und Reduktionsmittel liegen in diesen zweikomponentigen Systemen getrennt vor, wobei das Benzoylperoxid als Oxidationsmittel in der flüssigen, wäßrigen Phase zusammen mit der sauren Polyacrylsäure formuliert wurde. Es ist daher zu erwarten, daß das System keine große Lagerstabilität aufweist.

Dieser spezielle, durch eine Redoxreaktion initiierte, harzmodifizierte Glasionomerzement soll die Nachteile der herkömmlichen Glasionomerzemente wie Feuchtigkeitsempfindlichkeit und Bindungsstärke überwinden.

In der EP 1 269 967 A1 wird ein Kit beansprucht, das einen Primer sowie einen harzmodifizierten Glasionomerzement, der mit Hilfe einer Redoxreaktion gestartet wird, umfaßt.

Die EP 0 951 896 betrifft auch chemisch härtende Dentalwerkstoffe auf Basis zweikomponentiger, kunststoffmodifizierter Glasionomerzemente in der Form Pulver/Flüssigkeit, in denen der Füllstoff mit dem Oxidationsmittel und dem Reduktionsmittel beschichtet ist. Es wird angegeben, daß zusätzlich Beschleuniger wie Metallsalze in der festen Phase vorhanden sein können. Die Materialien sollen als Zemente oder Füllungswerkstoffe geeignet sein.

Die US 2003/0166740 A1 beschreibt ebenfalls chemisch härtende, zweikomponentige, kunststoffmodifizierte Glasionomerzemente in der Form Pulver/Flüssigkeit und Paste/Paste, die ohne Konditionierung an der Zahnhartsubstanz aufgebracht werden können. Die Palette tauglicher Reduktionsmittel wird hier um polymerisationsfähige Harnstoff- und Thioharnstoffverbindungen erweitert. Es wird ausgeführt (in Paragraph [0077]), daß in solchen Systemen Reduktions- und Oxidationsmittel in getrennten Teilen zu formulieren sind. Man kann sie gemeinsam in einen Teil geben, falls man die Technik der Mikroverkapselung anwendet (siehe hierzu auch die US 5,154,762). Das Dokument gibt auch Haftwerte der Zusammensetzungen an Rinderdentin an. In der Form Paste/Paste wurden Werte von 2, 8 bis 4,0 MPa gefunden (Tabelle 6).

In der US 2003/0195273 A1 werden chemisch härtende, zweikomponentige, kunststoffmodifizierte Glasionomerzemente offenbart. Die Formulierungen in der Form Paste/Paste sollen durch Zugabe von Salzen entscheidend in ihren Lagerstabilitäten verbessert werden. Die Haftwerte an Rinderdentin liegen zwischen 1,8 und 3,5 MPa.

Es war daher die Aufgabe der vorliegenden Erfindung, ein selbstadhäsives, lagerstabiles System auf der Grundlage eines kunstoffmodifizierten Glasionomerzementes zur Befestigung, zur Unterfüllung sowie zur Füllung bereitzustellen, das verbesserte Haftwerte gegenüber dem Stand der Technik aufweist. Das Material sollte in der Form Pulver/Flüssigkeit formuliert sein, um vorrangig den Glasionomercharakter und die damit verbundenen Vorteile aufrechtzuerhalten und einfach sowohl in seiner Herstellung als auch in seiner Anwendung sein.

Wie aus dem Stand der Technik hervorgeht, sind bei der Herstellung kunststoffmodifizierter Glasionomerzemente das Reduktionsmittel und das Oxidationsmittel in besonderer Weise und damit aufwendig zu behandeln, falls beide zusammen im Pulverteil eines solchen Materials vorliegen. Die Redoxpartner sollten entweder durch Mikroverkapselung voneinander getrennt werden, oder durch Beschichtung auf den Füllstoff an ein Substrat gebunden sein.

Es wurde nun überraschenderweise gefunden, daß die erfindungsgemäßen Formulierungen Haftwerte an Rinderdentin von 6 - 8 MPa, an Komposit von 12 - 13 MPa und an Keramik von 11 - 12 MPa erreichen, ohne daß ein vorbehandelnder Schritt erfolgen muß. Die erfindungsgemäßen Formulierungen weisen zudem gute mechanische Eigenschaften auf und sind einfach zu handhaben, wobei ein gleichbleibend stabiles Eigenschaftsprofil der Zemente bei Mischungsverhältnissen von Pulver zu Flüssigkeit bei den jeweiligen Indikationen vorgefunden wird. Für Befestigungen wird ein Mischungsverhältnis Pulver zu Flüssigkeit (P/F) zwischen 1,4 und 2,0, für Unterfüllungen zwischen 2,1 und 2,5 und für Füllungen zwischen 2,1 und 2,8 eingestellt.

Darüberhinaus erweist sich die Herstellung der erfindungsgemäßen Materialien als erfreulich einfach. Die Redoxpartner brauchen weder verkapselt noch an den Füllstoff gebunden werden.

Überdies wurde beobachtet, daß die Konsistenz der erfindungsgemäßen Zemente optimal der speziellen Anwendung angepaßt ist. Der Zement kann dünn beispielsweise auf einen Zahnstumpf aufgetragen werden. Wird dann eine Krone aufgesetzt, so quillt das Überschußmaterial aus dem Präparationsrand und bleibt dort standfest stehen. Der Anwender kann das Material in aller Ruhe entfernen. Zemente aus dem Stand der Technik fließen schnell am Zahn ab und können mit der Gingiva in Kontakt kommen.

Die erfindungsgemäßen, selbsthärtenden, dentalen Zusammensetzungen bestehen aus einem Pulverteil A und einem Flüssigkeitsteil B.

Der Pulverteil A enthält die Glaszusammensetzung a.), das Redoxsystem b.), Additive c.) und optional das Polymer einer α,β-ungesättigten Carbonsäure d.).

Der Flüssigkeitsteil B enthält das Polymer einer α,β-ungesättigten Carbonsäure d.), das Wasser e.), mindestens eine, eine ethylenisch ungesättigte Gruppe aufweisende Hydroxylverbindung f.), mindestens eine, zwei ethylenisch ungesättigte Gruppen aufweisende Hydroxylverbindung g.), mindestens ein, mindestens zwei ethylenisch ungesättigte Gruppen aufweisendes Monomer h.) und ebenfalls Additive c.).

Das Glaspulver a.), das erfindungsgemäß eingesetzt wird, unterliegt keinen besonderen Beschränkungen. Es muß in der Lage sein, mit dem Polymer einer α,β-ungesättigten Carbonsäure mit einem Gewichtsmittel des Molekulargewichtes zwischen 100 g/mol bis 120.000 g/mol, insbesondere zwischen 40.000 g/mol bis 100.000 g/mol, zu reagieren. Bevorzugt eingesetzt wird ein Strontiumaluminiumfluorsilikatglas, das auf seiner Oberfläche mit einer organischen Verbindung mit einer polymerisierbaren ethylenisch ungesättigten Doppelbindung in einer Menge von 0,1 bis 25 Gew.-% bezogen auf 100 Gew.-% des Glaspulvers überzogen ist. Durch die Oberflächenmodifizierung, die bevorzugt mit Methacryloxypropyltrimethoxysilan vorgenommen wird, werden die physikalischen Eigenschaften des resultierenden lonomers weiter verbessert. Das Glaspulver weist eine mittlere Teilchengröße von 0,2 µm bis 20 µm und eine Dichte von 2,2 bis 4,1 auf.

Das erfindungsgemäß eingesetzte Strontiumaluminiumfluorsilikatglas wird nach den bekannten Verfahren hergestellt. Beispielsweise verwendet man Siliziumdioxid, Aluminiumoxid, Strontiumsilikat, Natriumfluorid, Natriumaluminiumfluorid, Aluminiumphosphat und Strontiumphosphat. Das Rohmaterial wird gemischt und bei über 1000°C geschmolzen, abgekühlt und gemahlen.

Vorzugsweise macht das Strontiumaluminiumfluorsilikatglas a.) 30 bis 85 Gew.-% bezogen auf 100 Gew.-% der Gesamtzusammensetzung aus.

Das erfindungsgemäße Redoxsystem b.), das sicherstellt, daß der Zement chemisch unter sauren Bedingungen aushärtet und seine Eigenschaften in dem Bereich des Mischungsverhältnisses von Pulver zu Flüssigkeit von 1,4 bis 2,8 entsprechend seinen Indikationen stabil beibehält, umfaßt folgende Bestandteile:
b.1. 25 bis 80 Gew.-%, bevorzugt 35 bis 60 Gew.-% einer Barbitursäure oder Thiobarbitursäure bzw. einem Barbitursäure- oder Thiobarbitursäurederivat,
b.2. 25 bis 65 Gew.-%, bevorzugt 38 bis 57 Gew.-% einer Peroxodisulfat und/oder Peroxodiphosphatverbindung
b.3. 0,1 bis 8 Gew.-% einer Kupferverbindung
b.4. 0,05 bis 7 Gew.-% einer Verbindung mit einem ionogen vorliegendem Halogenatom,
wobei sich die Gewichtsteile auf 100 Gew.-% des Redoxsystems beziehen.

Unter dem Begriff "eine Verbindung" wird verstanden, daß mindestens eine Verbindung eingesetzt werden muß. Selbstverständlich können auch Mischungen geeigneter Verbindungen verwendet werden.

Beispielhafte Verbindungen der erfindungsgemäßen Komponente b.1. sind die Barbitursäure, die Thiobarbitursäure, die 1,3,5-Trimethylbarbitursäure, die 1-Phenyl-5-benzylbarbitursäure, die 1-Benzyl-5-phenylbarbitursäure, die 1,3-Dimethylbarbitursäure, die 1,3-Dimethyl-5-phenylbarbitursäure, usw.. Die erfindungsgemäßen Barbitursäure-, bzw. Thiobarbitursäurederivate weisen im Allgemeinen geeignete Substituenten an den Positionen 1, 3 und 5 des Ringstrukturelementes auf. Brauchbare Substituenten sind beispielsweise Alkyl-, substituierte Alkygruppen, Alkenyl-, Cycloalkyl- , substuituierte Cycloalkyl-, Arylalkyl-, Aryl- oder substituierte Arylgruppen. Die Substituenten können auch Halogenatome, Hydroxy-, Alkoxy-, Nitro-, oder Aminogruppen sein.

Beispielhafte Verbindungen der erfindungsgemäßen Komponente b.2. sind die salzartigen, wasserlöslichen Verbindungen wie Natriumperoxodisulfat, Ammoniumperoxodisulfat oder Kaliumperoxodisulfat sowie die analogen Peroxodiphosphatverbindungen. Bevorzugt verwendet wird das Natriumperoxodisulfat.

Beispielhafte Verbindungen der erfindungsgemäßen Komponente b.3. sind Kupferchlorid, Kupferacetat, Kupferacetylacetonat, Kupfernaphthenat, Kupfersalicylat, Komplexverbindungen des Kupfers wie die Komplexe von Kupfer mit Thioharnstoff oder Ethylendiamintetraessigsäure sowie das Bis-(1-Phenylpentan-1,3-dionato)-kupfer (11).

Beispielhafte Verbindungen der erfindungsgemäßen Komponente b.4. sind Verbindungen, in denen die ionogen gebundenen Halogene in Form ihrer löslichen Salze vorliegen, wie Dibutylammoniumchlorid, β-(Phenylethyl)-dibutylammoniumchlorid, Benzyltributylammoniumchlorid, Dilauryldimethylammoniumchlorid, Dioctyldimethylammoniumchlorid, usw.

Eine erfindungsgemäße Zusammensetzung des Redoxsystems besteht aus
b.1. 1-Benzyl-5-phenylbarbitursäure (48 Gew.-%)
b.2. Natriumperoxodisulfat (46% Gew.-%)
b.3. Kupferacetylacetonat (3 Gew.-%)
b.4. Benzyltributylammoniumchlorid (3 Gew.-%)

Alle Komponenten sind Feststoffe, liegen in der festen Phase vor und sind somit Bestandteile der Pulverkomponente der erfindungsgemäßen Zusammensetzung.

In der Gesamtzusammensetzung beträgt der mengenmäßige Anteil des Redoxsystems zwischen 0,05 und 2,50 Gew.-%.

Man kann vermuten, daß nach Aktivierung des Systems, also nach Mischen des Pulvers mit der Flüssigkeit, das Kupfer-(II)-ion die Enolatform der der 1-Benzyl-5-phenylbarbitursäure oxidiert, von dort ein Elektron abzieht und ein freies α-Carbonylradikal bildet, das durch zwei benachbarte Carbonylgruppen sowie einer Phenylgruppe stabilisiert ist. Das Peroxodisulfat bildet nun an der methylenaktiven Stelle des Reduktionsmittels ein Hydroperoxid, das die radikalische Polymerisation auslöst. Die Gegenwart des Benzyltributylammoniumchlorids soll lösliche Chloridionen zur Verfügung stellen und damit gewährleisten, daß schwerlösliche Kupfer-(I)-ionen, die bei der Redoxreaktion zwischen dem Kupferkomplex und dem Reduktionsmittel entstehen und nicht durch vorhandene chelatisierende Liganden zu komplexieren sind, nicht ausfallen, sondern durch Bildung von Spezies wie CuCl₂⁻, CuCl₃²⁻ in Lösung gehalten werden.

Zu den Additiven c.), die sowohl in den Flüssigkeitsteil A als auch in den Pulverteil B gegeben werden können, werden u.a. Farbmittel (anorganische und organische Pigmente), Mittel zur Kontrolle der Abbindereaktion, Modifikatoren zum Einstellen der rheologischen Eigenschaften, Füllstoffe, Stabilisatoren, Inhibitoren und bakterizid wirkende Substanzen gezählt.

Ein Mittel zur Kontrolle der Abbindereaktion ist beispielsweise die (+) Weinsäure, die die Verarbeitungszeit während der Glasionomerzementhärtung verlängert, die Abbindungsgeschwindigkeit jedoch gleichzeitig beschleunigt. Die Pulverkomponente der erfindungsgemäßen Zusammensetzung kann zusätzlich zum Glas noch einen anorganischen Füllstoff enthalten, wie er in den herkömmlichen dentalen Kompositmaterialien eingesetzt wird. Dieser Füllstoff weist in der Regel Teilchengrößen von 10 nm bis 20 µm auf und geht keine Reaktion mit dem Polymer einer α,β-ungesättigten Carbonsäure ein. Der anorganische Füllstoff kann beispielsweise Quarz, Siliziumdioxid, Titandioxid, Bariumsulfat, usw. sein.

Der Füllstoff kann auch organischer Natur sein. So können beispielsweise ein Polyethylmethacrylat oder andere gängige Copolymere einzeln oder mit dem anorganischen Füllstoff zusammen der erfindungsgemäßen Zusammensetzung beigegeben werden.

Der Anteil der Additive an der Gesamtzusammensetzung ist optional und besteht zwischen 0 und 15 Gew.-%.

Das Polymer einer α,β-ungesättigten Carbonsäure d.) entsteht im Verlauf einer radikalischen Polymerisation einer wäßrigen, α,β-ungesättigten Acryl-, Methacryl-, Itakon-, Fumar-, oder Maleinsäure. Werden die organischen Mono- oder Dicarbonsäuren einzeln polymerisiert, so erhält man Homopolymere. Wird die Polymerisation in Gegenwart von zwei oder mehreren Säuren durchgeführt, so führt dies zu Copolymeren.

Erfindungsgemäß werden ausschließlich Homo- oder Copolymere der Acryl- oder Maleinsäure in Form ihrer wäßrigen Lösung eingesetzt. Bevorzugt ist der Einsatz der Polyacrylsäure mit mittleren Molekulargewichten zwischen 40.000 g/mol und 100.000 g/mol. Die Polyacrylsäure ist so zu wählen, daß ihr Molekulargewicht und damit ihre Kettenlänge möglichst hoch ist. Ihr mengenmäßiger Anteil an der Gesamtzusammensetzung liegt zwischen 2 und 25 Gew.-%. Die Polyacrylsäure kann auch in den Pulverteil aufgenommen werden.

Der Anteil des Wassers an der erfindungsgemäßen Zusammensetzung beträgt zwischen 4 und 25 Gew.-%.

Beispiele für eine ethylenisch ungesättigte Gruppen aufweisende, radikalisch vernetzbare Hydroxylverbindung f.) sind 2-Hydroxyethylmethacrylat, 3-Hydroxypropylmethacrylat, 2-Hydroxypropylmethacrylat, 1,3-Dihydroxypropylmethacrylat, 2,3-Dihydroxypropylmethacrylat, Glyzerinmonomethacrylat, usw.. Der mengenmäßige Anteil der eine ethylenisch ungesättigte Gruppe aufweisenden Hydroxylverbindung beträgt zwischen 1,5 und 20 Gew.-% an der Gesamtzusammensetzung.

Beispiele für zwei ethylenisch ungesättigte Gruppen aufweisende, radikalisch vernetzbare Hydroxylverbindung g.) sind Glyzerindimethacrylat und 2,3-bis[4-[3-methacryloyloxy-2-hydroxypropoxy]phenyl]-propan. Ihr mengenmäßiger Anteil an der Gesamtzusammensetzung beträgt zwischen 0,2 und 9 Gew.-%.

Beispiele für ein mindestens zwei ethylenisch ungesättigte Gruppen aufweisendes Monomer sind die in Kompositen üblicherweise eingesetzten Monomeren, wie Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-5,12-diazahexadecan-1,16-dioxy-dimethacrylat, Butandioldimethacrylat, Trimethylolpropantrimethacrylat, Pentaerythrittetramethacrylat, Dipentaerythrithexamethacrylat, Tetraethylenglykoldimethacrylat, Neopentylglykoldimethacrylat, usw..

Weitere geeignete Monomere sind beispielsweise in der DE 39 41 629 A1 angegeben.

Ihr mengenmäßiger Anteil an der Gesamtzusammensetzung beträgt zwischen 0,1 und 5 Gew.-%.

Die Erfindung wird nachfolgend anhand von Beispielen näher beschrieben.

### Beschreibung der Herstellung der Pulverkomponente:

Die Pulverkomponente enthält den reaktiven Füllstoff, das Redoxsystem und optional die Additive. Die Komponenten werden in feinstgemahlener Form (Kugelmühle) durch intensives Homogenisieren vereinigt. Das intensive Homogenisieren lässt sich z.B. durch die Verwendung eines Taumelmischers durchführen.

### Beschreibung der Herstellung der Flüssigkeitskomponente:

Die Flüssigkeitskomponente enthält folgende Komponenten: die vernetzbaren Verbindungen, das Polymer einer α,β-ungesättigten Carbonsäure, Wasser und optional die Additive. Diese Komponenten werden in obiger Reihenfolge intensiv homogenisiert.

Das eingesetzte Redoxsystem entspricht der erfindungsgemäßen Zusammensetzung von Seite 13 des Beschreibungsteils. Als Inhibitor wurde das BHT (3,5-di-tert.butyl-4-hydroxytoluol) eingesetzt.

### Beschreibung der Durchführung der Haftungstests:

Die Präparation der Prüfkörper verläuft wie folgt: Ein Prüfkörper aus Rinderdentin wird auf einem Nassschleifblock beschliffen. Die Dentinfläche wird mit einem Luftbläser derart getrocknet, dass ein dünner Feuchtigkeitsfilm zurückbleibt. Auf der so präparierten Dentinfläche wird eine perforierte Gummischeibe platziert. Das erfindungsgemäße Material wird in den entsprechenden Mischungsverhältnissen auf einer Glasplatte eingewogen und anschließend mit einem entsprechenden Mischspatel für 30s homogen gemischt. Die Mischung wird in der perforierten Gummischeibe platziert. Anschließend wird der Prüfkörper für 24 Stunden bei 37°C und 100% rel. Luftfeuchtigkeit gelagert.

Die Haftwerte werden mittels einer Universalprüfmaschine gemessen, die die Prüfung gem. eines Parametersatzes durchführt (Vorschubgeschwindigkeit: 1 mm/min). Hierfür wird der Dentinprüfkörper in einen Scherblock eingespannt, der mit einem Scherkeil versehen ist. Für die Prüfung muß der Scherkeil leichtgängig sein und darf nicht verkanten. Nach diesen Vorbereitungen wird die Prüfung durchgeführt. Die Haftwerte ergeben sich aus dem Quotienten der Multiplikation der Summe der gemessenen Kraft F und der Gewichtskraft des Scherkeils (1,3 N) mit einem Flächenfaktor (1,27) und dem Produkt des an zwei Meßpunkten ermittelten Durchmessers des abgescherten Prüfkörpers. Die Auswertung erfolgt automatisch.

Die Abkürzung P/L (powder/liquid) bedeutet das Mischungsverhältnis von Pulver zur Flüsssigkeit.

**Beispiel 1**

| Flüssigkeit | Anteil [%] | Pulver | Anteil [%] |
|---|---|---|---|
| Polyacrylsäure | 24,0 | Strontiumaluminiumfluorsilikatglas | 93,0 |
| Hydroxyethylmethacrylat | 30,9 | Polyacrylsäure | 6,0 |
| Glyzerindimethacrylat | 5,0 | Redoxsystem | 1,0 |
| Urethandimethacrylat | 2,0 | | |
| Wasser | 36,0 | | |
| Weinsäure | 2,0 | | |
| Inhibitor | 0,1 | | |

**Beispiel 2**

| Flüssigkeit | Anteil [%] | Pulver | Anteil [%] |
|---|---|---|---|
| Polyacrylsäure | 30,0 | Strontiumaluminiumfluorsilikatglas | 99,0 |
| Wasser | 30,0 | Redoxsytem | 1,0 |
| Hydroxyethylmethacrylat | 31,9 | | |
| Glyzerindimethacrylat | 4,0 | | |
| Urethandimethacrylat | 2,0 | | |
| Weinsäure | 2,0 | | |
| Inhibitor | 0.1 | | |

**Beispiel 3**

| Flüssigkeit | Anteil [%] | Pulver | Anteil [%] |
|---|---|---|---|
| Polyacrylsäure | 30,0 | Strontiumaluminiumfluorsilikatglas | 97,9 |
| Wasser | 30,0 | Redoxsystem | 2,1 |
| Hydroxyethylmethacrylat | 20,9 | | |
| Glyzerindimethacrylat | 13,0 | | |
| Urethandimethacrylat | 4,0 | | |
| Weinsäure | 2,0 | | |
| Inhibitor | 0,1 | | |

**Beispiel 4**

| Flüssigkeit | Anteil [%] | Pulver | Anteil [%] |
|---|---|---|---|
| Polyacrylsäure | 28,0 | Strontiumaluminiumfluorsilikatglas | 99,0 |
| Wasser | 26,5 | Redoxsystem | 1,0 |
| Hydroxyethylmethacrylat | 26,9 | | |
| Glyzerindimethacrylat | 12,0 | | |
| Urethandimethacrylat | 4,0 | | |
| Weinsäure | 2,5 | | |
| Inhibitor | 0,1 | | |

**Beispiel 5**

| Flüssigkeit | Anteil [%] | Pulver | Anteil [%] |
|---|---|---|---|
| Polyacrylsäure | 27,5 | Strontiumaluminiumfluorsilikatglas | 97,5 |
| Wasser | 31,5 | Redoxsystem | 2,5 |
| Hydroxyethylmethacrylat | 19,0 | | |
| Glyzerindimethacrylat | 11,9 | | |
| Urethandimethacrylat | 8,0 | | |
| Inhibitor | 0,1 | | |
| Weinsäure | 2,0 | | |

**Beispiel 6**

| Flüssigkeit | Anteil [%] | Pulver | Anteil [%] |
|---|---|---|---|
| Polyacrylsäure | 30,0 | Strontiumaluminiumfluorsilikatglas | 98,7 |
| Wasser | 27,0 | Redoxsystem | 1.3 |
| Hydroxyethylmethacrylat | 21,5 | | |
| Glyzerindimethacrylat | 13,4 | | |
| Urethandimethacrylat | 4,9 | | |
| Weinsäure | 3,0 | | |
| Inhibitor | 0,2 | | |

**Beispiel 7**

| Flüssigkeit | Anteil [%] | Pulver | Anteil [%] |
|---|---|---|---|
| Polyacrylsäure | 29,5 | Strontiumaluminiumfluorsilikatglas | 98,3 |
| Wasser | 29,5 | Redoxsystem | 1,7 |
| Hydroxyethylmethacrylat | 22,4 | | |
| Glyzerindimethacrylat | 11,5 | | |
| Urethandimethacrylat | 4,0 | | |
| Inhibitor | 0,1 | | |
| Weinsäure | 3,0 | | |

**Beispiel 8**

| Flüssigkeit | Anteil [%] | Pulver | Anteil [%] |
|---|---|---|---|
| Polyacrylsäure | 25,0 | Strontiumaluminiumfluorsilikatglas | 98,5 |
| Wasser | 30,0 | Redoxsystem | 1,5 |
| Hydroxyethylmethacrylat | 26,4 | | |
| Glyzerindimethacrylat | 11,5 | | |
| Urethandimethacrylat | 4,0 | | |
| Weinsäure | 3,0 | | |
| Inhibitor | 0,1 | | |

**Beispiel 9**

| Flüssigkeit | Anteil [%] | Pulver | Anteil [%] |
|---|---|---|---|
| Polyacrylsäure | 28,0 | Strontiumaluminiumfluorsilikatglas | 98,2 |
| Wasser | 30,0 | Redoxsystem | 1,8 |
| Hydroxyethylmethacrylat | 22,0 | | |
| Glyzerindimethacrylat | 12,5 | | |
| Urethandimethacrylat | 5,5 | | |
| Weinsäure | 1,9 | | |
| Inhibitor | 0,1 | | |

**Beispiel 10**

| Flüssigkeit | Anteil [%] | Pulver | Anteil [%] |
|---|---|---|---|
| Polyacrylsäure | 35,0 | Strontiumaluminiumfluorsilikatglas | 99,0 |
| Wasser | 35,4 | Redoxsystem | 1,0 |
| Hydroxyethylmethacrylat | 13,0 | | |
| Glycerindimethacrylat | 8,0 | | |
| Urethandimethacrylat | 6,0 | | |
| Inhibitor | 0,1 | | |
| Weinsäure | 2,5 | | |

**Haftung am Dentin, P/L= 2,0:1**

| Beispiel | Haftung [MPa] | DH [MPa] |
|---|---|---|
| 1 | 5,8 | 93,1 |
| 2 | 7,2 | 82,4 |
| 3 | 6,8 | 90,3 |
| 4 | 8,4 | 116,2 |
| 5 | 6,2 | 93,2 |
| 6 | 5,7 | 100,9 |
| 7 | 8,1 | 91,7 |
| 8 | 7,4 | 101,6 |
| 9 | 6,4 | 113,4 |
| 10 | 7,9 | 99,3 |

**Beispiel 4, Haftung an Komposit**

| | |
|---|---|
| | Haftung [MPa] |
| Komposit Lichthärtung | 13,0¹⁾ |
| Komposit chem. Härtung | 11,9¹⁾ |

| | |
|---|---|
| ¹⁾kohäsiver Bruch | |

Als Kompositmaterial wurde das VOCO Produkt Rebilda LC (lichtgehärtet) und Rebilda DC (chemisch gehärtet) benutzt.

**Beispiel 4, Haftung an Dentin**

| P/L-Verhältnis | Haftung [MPa] | DH4x6 [MPa] |
|---|---|---|
| 1,6:1 | 7,6 | 84,2 |
| 1,7:1 | 7,8 | 89,3 |
| 1,8:1 | 8,1 | 92,5 |
| 1,9:1 | 7,8 | 96,4 |
| 2,0:1 | 8,4 | 116,2 |
| 2,2:1 | 6,9 | 118,9 |
| 2,5:1 | 7,1 | 145,6 |
| 2,8:1 | 5,8 | 148,7 |

Die vorstehenden Daten beziehen sich auf die Haftung an Rinderdentin als Substrat. In der klinischen Praxis werden Restaurationen aus verschiedenen Materialien verwendet, so dass Haftungsuntersuchungen an entsprechenden Brücken- und Kronenmaterialien ebenfalls von Bedeutung sind. Im Folgenden ist eine solche Untersuchung der Haftung des Materials an Zirkondioxid mit Beschreibung der Testvorbereitung und des Testablaufs aufgeführt:

### Prüfverfahren:

Prüfverfahren nach LANGE, VÖLKL, ERTL (Degudent, Hannau 2004)
Prüfanordnung nach GEIS-GERSTORFER (Universität Tübingen 2005)

*Messung der Bruchfestigkeit bei statischer Druck-*/*Scherbelastung:* Das Haftverbundsystem besteht aus einer Zementfuge definierten Ausmaßes (52 mm²) des nach Herstellerangaben verarbeiteten Zementes zwischen ROCTEC^{®} -konditionierten und ESPE^{®}-Sil-silanisierten Zirkoniumdioxid-Oberflächen.

### Lagerungsmodi:

*Initial:* 24h - Lagerung in destilliertem Wasser
*Thermozyklisch:* 15.000 Mal Wechsel zwischen 55°C warmem Wasser (Haltezeit: 30s), danach 10s Tropfpause, und 5°C kaltem Wasser (Haltezeit: 30s), danach 10s Tropfpause

### Zementierlasten:

Die Prüfkörper wurden mit den entsprechenden Mischungen unterschiedlichen Zementierlasten ausgesetzt, um den Einfluss der Kraft, die der Anwender beim Einsetzen einer Restauration aufbringt, auf die Haftung zu untersuchen. Es wurden 4 verschiedene Lasten getestet: 2, 4, 6 und 8kg.

Die Ergebnisse dieser Untersuchungen sind in den folgenden Tabellen dargestellt:

**Beispiel 4, Haftung an Zirkoniumdioxid**

| P/L-Verhältnis | Haftung [MPa], 24h | Haftung [MPa], 24h, Thermocycling |
|---|---|---|
| 1,0:1 | 7,0 | 5,0 |
| 1,2:1 | 8,9 | 13,2 |
| 1,4:1 | 11,9 | 10,3 |
| 1,6:1 | 11,5 | 13,8 |
| 1,8:1 | 12,3 | 12,9 |
| 2,0:1 | 11,1 | 12,3 |

| | | |
|---|---|---|
| **24h:** Lagerung des Probekörpers in dest. Wasser, Thermocycling: 15.000 [55°C (30") - Tropfpause (10") - 5°C (30")] | | |

**Beispiel 4, P/L-Verhältnis: 1,6:1, Haftung an Zirkoniumdioxid**

| Zementierlast (kg) | Haftung [MPa], 24h | Haftung [MPa], 24h Thermocycling |
|---|---|---|
| 2 | 10,0 | 14,0 |
| 4 | 11,1 | 12,8 |
| 6 | 10,1 | 14,3 |
| 8 | 11,5 | 11,5 |

| | | |
|---|---|---|
| **24h:** Lagerung des Probekörpers in dest. Wasser, **Thermocycling:** 15.000 [55°C (30") - Tropfpause (10") - 5°C (30")] | | |

**Beispiel 4, P/L-Verhältnis: 1,8:1, Haftung an Zirkoniumdioxid**

| Zementierlast (kg) | Haftung [MPa], 24h | Haftung [MPa], 24h Thermocycling |
|---|---|---|
| 2 | 10,4 | 14,0 |
| 4 | 10,8 | 12,8 |
| 6 | 11,0 | 14,3 |
| 8 | 11,5 | 11,5 |

## Patentansprüche

1. Selbsthaftendes, zweikomponentiges Pulver/Flüssigkeit Dentalmaterial, umfassend
A. einen Pulverteil, der enthält
A.a. 30 bis 85 Gew.-% einer basische Glaszusammensetzung, die in der Lage ist, mit einem Polymer einer α,β-ungesättigten Carbonsäure zu reagieren
A.b. 0,05 bis 2,50 Gew.-% eines Redoxsytems, das enthält
A.b.1. 25 bis 80 Gew.-% einer Barbitursäure oder Thiobarbitursäure bzw. eines Barbitursäure- oder Thiobarbitursäurederivats
A.b.2. 25 bis 65 Gew.-% einer Peroxodisulfat- und/oder Peroxodiphosphatverbindung
A.b.3. 0,1 bis 8 Gew.-% einer Kupferverbindung
A.b.4 0,05 bis 7 Gew.-% einer Verbindung mit einem ionogen vorliegendem Halogenatom,
wobei sich die Gewichtsteile auf 100 Gew.-% des Redoxsystems beziehen,
A.c. 0 bis 15 Gew.-% Additive wie Farbmittel Weinsäure, Kieselsäure, Füllstoffe, Stabilisatoren, Inhibitoren und bakterizid wirkende Substanzen,
sowie
B. einen Flüssigkeitsteil, der enthält
B.a. 2 bis 18 Gew.-% eines Polymers einer α,β-ungesättigten Carbonsäure
B.b. 2 bis 25 Gew.-% Wasser
B.c. 1,5 bis 20 Gew.-% einer, eine ethylenisch ungesättigte Gruppe aufweisende Hydroxylverbindung
B.d. 0,2 bis 9 Gew.-% einer, zwei ethylenisch ungesättigte Gruppen aufweisende Hydroxylverbindung
B.e. 0,1 bis 5 Gew.-% eines, mindestens zwei ethylenisch ungesättigte Gruppen aufweisenden Monomers sowie
A.c. 0 bis 7 Gew.-% Additive wie Farbmittel, Weinsäure, Kieselsäure, Füllstoffe, Stabilisatoren, Inhibitoren und bakterizid wirkende Substanzen, wobei sich die Gew.-% auf 100 Teile der Gesamtzusammensetzung beziehen.

2. Dentalmaterial nach Anspruch 1, **dadurch gekennzeichnet, daß** das Mischungsverhältnis des Pulverteils A zum Flüssigkeitsteil B zwischen 1,4 und 2,8 beträgt.

3. Dentalmaterial nach Anspruch 1, **dadurch gekennzeichnet, daß** das Redoxsystem A.b. die Bestandteile
A.b.1. zu 35 bis 60 Gew.-%
A.b.2. zu 38 bis 57 Gew.-%
enthält.

4. Dentalmaterial nach einem der obigen Ansprüche, **dadurch gekennzeichnet, daß** das Redoxsystem A.b. als Bestandteile
A.b.1 die 1-Benzyl-5-phenylbarbitursäure
A.b.2. das Natriumperoxodisulfat
A.b.3. das Kupferacetylacetonat und
A.b.4. das Benzyltributylammoniumchlorid
enthält.

5. Dentalmaterial nach einem der vorstehenden Ansprüche, **dadurch gekennzeichnet, daß** das Polymer einer α,β-ungesättigten Carbonsäure B.a. ein mittleres Molekulargewicht zwischen 40.000 und 100.000 g/mol aufweist.

6. Dentalmaterial nach einem der vorstehenden Ansprüche, **dadurch gekennzeichnet, daß** die basische Glaszusammensetzung A.a. eine mittlere Teilchengöße von 0,2 µm bis 20 µm aufweist.

7. Dentalmaterial nach einem der vorstehenden Ansprüche, **dadurch gekennzeichnet, daß** die basische Glaszusammensetzung ein Strontiumaluminiumfluorsilikatglas ist.

8. Dentalmaterial nach einem der vorstehenden Ansprüche, **dadurch gekennzeichnet, daß** die basische Glaszusammensetzung oberflächenmodifiziert ist.

9. Dentalmaterial nach Anspruch 8, **dadurch gekennzeichnet, daß** die Oberfläche der basischen Glaszusammensetzung mit einer organischen Verbindung mit einer polymerisierbaren ethylenisch ungesättigten Doppelbindung in einer Menge von 0,1 bis 25 Gew.-% bezogen auf 100 Gew.-% des Glaspulvers überzogen ist.

10. Dentalmaterial nach Anspruch 9, **dadurch gekennzeichnet, daß** die organische Verbindung mit einer polymerisierbaren ethylensich ungesättigten Doppelbindung das Methacryloxypropyltrimethoxysilan ist.

11. Selbsthaftendes, zweikomponentiges Pulver/Flüssigkeit Dentalmaterial nach einem der vorstehenden Ansprüche zur Verwendung als Befestigungsmaterial dentaler und kieferorthopädischer Vorrichtungen, Überkappungsmaterial der Pulpa, Unterfüllungsmaterial unter allen Füllungsmaterialien sowie als Füllungsmaterial restaurationsbedüftiger Zähne.

## Claims

1. Self-adhering two-component powder/liquid dental material, comprising
A. a powder part, which comprises
A.a. from 30 to 85% by weight of a basic glass composition which is capable of reacting with a polymer of an α,β-unsaturated carboxylic acid
A.b. from 0.05 to 2.50% by weight of a redox system, which comprises
A.b.1. from 25 to 80% by weight of a barbituric acid or thiobarbituric acid or of a barbituric acid or thiobarbituric acid derivative
A.b.2. from 25 to 65% by weight of a peroxodisulphate and/or peroxodiphosphate compound
A.b.3. from 0.1 to 8% by weight of a copper compound
A.b.4. from 0.05 to 7% by weight of a compound having a halogen atom existing in ionogenic form, the parts by weight being with reference to 100% by weight of the redox system,
A.c. from 0 to 15% by weight of additives, such as colourants, tartaric acid, silica, fillers, stabilizers, inhibitors and bactericidal substances,
and
B. a liquid part, which comprises
B.a. from 2 to 18% by weight of a polymer of an α,β-unsaturated carboxylic acid
B.b. from 2 to 25% by weight of water
B.c. from 1.5 to 20% by weight of a hydroxyl compound exhibiting an ethylenically unsaturated group
B.d. from 0.2 to 9% by weight of a hydroxyl compound exhibiting two ethylenically unsaturated groups
B.e. from 0.1 to 5%. by weight of a monomer exhibiting at least two ethylenically unsaturated groups,
A.c. from 0 to 7% by weight of additives, such as colorants, tartaric acid, silica, fillers, stabilizers, inhibitors and bactericidal substances,
the % by weight being with reference to 100 parts of the total composition.

2. Dental material according to Claim 1, **characterized in that** the mixing ratio of the powder part A to the liquid part B is between 1.4 and 2.8.

3. Dental material according to Claim 1, **characterized in that** the redox system A.b comprises the constituents:
A.b.1. at 35 to 60% by weight
A.b.2. at 38 to 57% by weight.

4. Dental material according to one of the preceding claims, **characterized in that** the redox system A.b. comprises, as constituents:
A.b.1. 1-benzyl-5-phenylbarbituric acid
A.b.2. sodium peroxodisulphate
A.b.3. copper acetylacetonate and
A.b.4. benzyltributylammonium chloride.

5. Dental material according to one of the preceding claims, **characterized in that** the polymer of an α,β-unsaturated carboxylic acid B.a. exhibits an average molecular weight between 40 000 and 100 000 g/mol.

6. Dental material according to one of the preceding claims, **characterized in that** the basic glass composition A.a. exhibits a mean particle size of 0.2 µm to 20 µm.

7. Dental material according to one of the preceding claims, **characterized in that** the basic glass composition is a strontium aluminium fluorosilicate glass.

8. Dental material according to one of the preceding claims, **characterized in that** the basic glass composition is surface-modified.

9. Dental material according to Claim 8, **characterized in that** the surface of the basic glass composition is coated with an organic compound having a polymerizable ethylenically unsaturated double bond in an amount of 0.1 to 25% by weight, with reference to 100% by weight of the glass powder.

10. Dental material according to Claim 9, **characterized in that** the organic compound having a polymerizable ethylenically unsaturated double bond is methacryloyloxypropyltrimethoxysilane.

11. Self-adhering two-component powder/liquid dental material according to one of the preceding claims for use as fixing material for dental and orthodontic devices, pulp capping material, filling material for insertion between the cavity base and all filling materials, and filling material for teeth in need of restoration.

## Revendications

1. Matériau dentaire bicomposant poudre/liquide autoadhésif, comprenant
A. une partie poudre, qui contient
A.a 30 à 85 % en poids d'une composition de verre basique qui est apte à réagir avec un polymère d'un acide carboxylique α,β-insaturé
A.b. 0,05 à 2,50 % en poids d'un système redox qui contient
A.b.1. 25 à 80 % en poids d'un acide barbiturique ou thiobarbiturique ou d'un dérivé d'acide barbiturique ou thiobarbiturique
A.b.2. 25 à 65 % en poids d'un composé peroxodisulfate et/ou peroxodiphosphate
A.b.3. 0,1 à 8 % en poids d'un composé à base de cuivre
A.b.4. 0,05 à 7 % en poids d'un composé comportant un atome d'halogène présent sous forme ionogène,
les parties en poids se rapportant à 100 % en poids du système redox,
A.c. 0 à 15 % en poids d'additifs tels que des colorants, l'acide d-tartrique, l'acide silicique, des charges, des stabilisants, des inhibiteurs et des substances à action bactéricide,
ainsi que
B. une partie liquide, qui contient
B.a. 2 à 18 % en poids d'un polymère d'un acide carboxylique α,β-insaturé
B.b. 2 à 25 % en poids d'eau
B.c. 1,5 à 20 % en poids d'un composé hydroxylé comportant un groupe à insaturation éthylénique
B.d. 0,2 à 9 % en poids d'un composé hydroxylé comportant deux groupes à insaturation éthylénique
B.e. 0,1 à 5 % en poids d'un monomère comportant au moins deux groupes à insaturation éthylénique ainsi que A.c. 0 à 7 % en poids d'additifs, tels que des colorants, l'acide d-tartrique, l'acide silicique, des charges, des stabilisants, des inhibiteurs et des substances à action bactéricide,
les % en poids se rapportant à 100 parties en poids de la composition totale.

2. Matériau dentaire selon la revendication 1, **caractérisé en ce que** le rapport de mélange de la partie poudre A à la partie liquide B est compris entre 1,4 et 2,8.

3. Matériau dentaire selon la revendication 1, **caractérisé en ce que** le système redox A.b. contient les composants
A.b.1. à raison de 35 à 60 % en poids
A.b.2. à raison de 38 à 57 % en poids.

4. Matériau dentaire selon l'une quelconque des revendications ci-dessus, **caractérisé en ce que** le système redox A.b. contient comme composants
A.b.1. l'acide 1-benzyl-5-phénylbarbiturique
A.b.2. le peroxodisulfate de sodium
A.b.3. l'acétylacétonate de cuivre et
A.b.4. le chlorure de benzyltributylammonium.

5. Matériau dentaire selon l'une quelconque des revendications précédentes, **caractérisé en ce que** le polymère d'un acide carboxylique α,β-insaturé B.a. présente une masse moléculaire moyenne comprise entre 40 000 et 100 000 g/mole.

6. Matériau dentaire selon l'une quelconque des revendications précédentes, **caractérisé en ce que** la composition de verre basique A.a. présente une taille moyenne de particule de 0,2 µm à 20 µm.

7. Matériau dentaire selon l'une quelconque des revendications précédentes, **caractérisé en ce que** la composition de verre basique est un verre fluorosilicate de strontium et d'aluminium.

8. Matériau dentaire selon l'une quelconque des revendications précédentes, **caractérisé en ce que** la composition de verre basique est modifiée en surface.

9. Matériau dentaire selon la revendication 8, **caractérisé en ce que** la surface de la composition de verre basique est revêtue avec un composé organique comportant une double liaison polymérisable à insaturation éthylénique, en une quantité de 0,1 à 25 % en poids, par rapport à 100 % en poids de la poudre de verre.

10. Matériau dentaire selon la revendication 9, **caractérisé en ce que** le composé organique comportant une double liaison polymérisable à insaturation éthylénique est le méthacryloxypropyltriméthoxysilane.

11. Matériau dentaire bicomposant poudre/liquide autoadhésif selon l'une quelconque des revendications précédentes, pour utilisation en tant que matériau de fixation d'appareils dentaires et d'orthodontie, matériau de coiffage de la pulpe, matériau de doublage en particulier matériaux d'obturation ainsi que comme matériau d'obturation de dents nécessitant une restauration.
